# EUROPÄISCHE PATENTSCHRIFT

(11) **EP 1 713 800 B1**
(45) Veröffentlichungstag und Bekanntmachung des Hinweises auf die Patenterteilung: **18.11.2009**
(21) Anmeldenummer: 05701061.3
(22) Anmeldetag: 20.01.2005
(51) Int. Cl.: C07D 451/02

(54) **VERFAHREN ZUR HERSTELLUNG EINES 2- (ETHOXYMETHYL)-TROPANDERIVATES**
METHOD FOR PRODUCING A 2-(ETHOXYMETHYL)TROPANE DERIVATIVE
PROCEDE POUR PRODUIRE UN DERIVE DE 2-(ETHOXYMETHYL)-TROPANE

(30) Priorität: 31.01.2004 DE 102004004965
(43) Veröffentlichungstag der Anmeldung: 25.10.2006
(73) Patentinhaber: NeuroSearch A/S, 2750 Ballerup (DK)
(72) Erfinder: EHLENZ, Richard, 55545 Bad Kreuznach (DE); MEYER, Oliver, 55452 Dorsheim (DE); WAGNER, Sascha, 55546 Frei-Laubersheim (DE)
(74) Vertreter: Abildgren, Michael Padkjaer
(86) Internationale Anmeldenummer: PCT/EP2005/000512
(87) Internationale Veröffentlichungsnummer: WO 2005/073228

(56) Entgegenhaltungen:
- WO-A-97/30997
- WO-A-02/102801
- WO-A-03/045388

## Beschreibung

Die vorliegende Erfindung betrifft ein verbessertes Verfahren zur Herstellung eines 2-(Ethoxymethyl)-tropanderivates durch Umsetzung eines 2-(Hydroxymethyl)-tropanderivates mit Ethylbromid in Gegenwart einer spezifischen Base und eines Phasentransferkatalysators.

2-(Ethoxymethyl)-tropanderivate sind wertvolle pharmazeutische Wirkstoffe zur Behandlung verschiedener zentralnervöser Störungen, wie z.B. Parkinsonismus oder der Alzheimer Krankheit.

Nach der Lehre der Veröffentlichung WO 97/30997 werden diese entweder aus einem 2-(Tosylmethyl)-tropanderivat durch Umsetzung mit Ethanolat oder durch Umsetzung eines 2-(Hydroxymethyl)-tropanderivates mit Natriumhydrid als Base und Diethylsulfat hergestellt. Aus Sicherheitsgründen ist eine Produktion im technischen Maßstab unter Einsatz von Natriumhydrid kaum durchführbar. Des Weiteren ist diese Ethylierung kaum reproduzierbar, verläuft mit langen Reaktionszeiten und ergibt den Wirkstoff als schwer isolierbaren Feststoff in unbefriedigenden Ausbeuten.

Die Veröffentlichung WO 02/102801 beschreibt Tropanderivate, wie z.B. 2-(Ethoxymethyl)-tropanderivate, die als Hemmer zur Wiederaufnahme von Monoamin-Neurotransmittern verwendet werden können.

Der vorliegenden Erfindung liegt die Aufgabe zu Grunde, ein Verfahren aufzuzeigen, welches es ermöglicht, 2-(Ethoxymethyl)-tropanderivate in guten Ausbeuten im großtechnischen Maßstab herzustellen und dabei die bei aus dem Stand der Technik bekannten Verfahren auftretenden Nachteile zu vermeiden.

Überraschenderweise wurde nun gefunden, dass 2-(Ethoxymethyl)-tropanderivate Formel (I) oder einem pharmazeutisch akzeptable Salze davon, 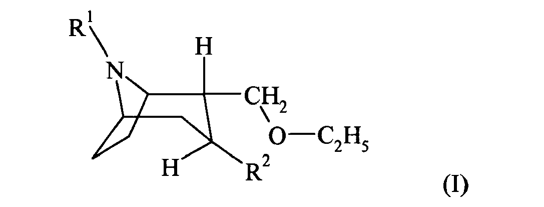 worin
R¹ für Wasserstoff oder C₁₋₆ Alkyl, insbesondere Methyl steht; und
R² für gegebenenfalls ein oder mehrfach durch Halogen, Trifluormethyl oder Cyano substituiertes Phenyl, insbesondere 3,4-Dichlorphenyl steht; in guten Ausbeuten und im technischen Maßstab durch Umsetzung eines 2-(Hydroxymethyl)-tropanderivates der Formel (II), 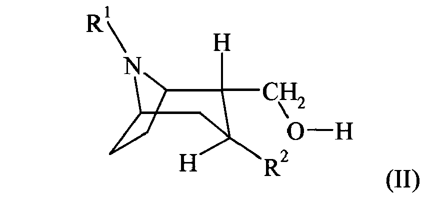
worin R¹ und R² die für Formel (I) angegebene Bedeutung aufweisen,
mit Ethylbromid in Gegenwart einer spezifischen Base, eines Phasentransferkatalysators, und gegebenenfalls einem Verdünnungsmittel hergestellt werden kann.

Gegenstand der Erfindung ist somit ein verbessertes Verfahren zur Herstellung eines 2-(Ethoxymethyl)-tropanderivates der Formel (I) oder einem pharmazeutisch akzeptablen Salz davon, wobei man ein 2-(Hydroxymethyl)-tropanderivate der Formel (II) mit Ethylbromid in Gegenwart einer Base, ausgewählt aus Lithiumhydroxid, Natriumhydroxid und Kaliumhydroxid, eines Phasentransferkatalysators, und gegebenenfalls einem Verdünnungsmittel umsetzt, und anschließend gegebenenfalls mit einer Säure behandelt.

Bevorzugte Ausführungsformen des erfindungsgemäßen Verfahren sind Verfahren, wobei:
(A) man als Base ein gepulvertes Kaliumhydroxid einsetzt;
(B) man als Phasentransferkatalysator (PTK) ein Tetraalkylammonium- oder Tetraalkylphosphoniumsalz, wobei die jeweiligen Alkylgruppen gleich oder verschieden sein können, wie zum Beispiel Salze von Tetraoktylammonium, Methyltrioktylammonium, Tetramethylammonium, Tetraethylammonium, Tetrahexylammonium Aliquat 175 (Tributylmethylammonium) oder Aliquat 336 (Methyltrioktylammonium) verwendet. Vorzugsweise ist der PTK ein Tetraalkylammonium Halogenid, ein Tetraalkylammonium Sulfat, ein Tetraalkylammonium Hydrogensulfat, ein Tetraalkylammonium Nitrat oder ein Tetraalkylammonium Phosphat, insbesondere ein Tetraalkylammonium Hydrogensulfat, ganz besonders bevorzugt Tetra-n-butylammonium Hydrogensulfat.

Der Begriff "Alkyl" wie er vor- und nachstehend im Bezug auf den Phasentransferkatalysator verwendet wird umfasst geradkettige und verzweigte Alkylgruppen mit 1 bis 8, vorzugsweise 2 bis 6, insbesondere 4 Kohlenstoffatomen. Als bevorzugte Alkylgruppen seien somit die Ethyl-, *n*-Propyl-, *i*-Propyl-, *n*-Butyl-, 2-Butyl, *tert*-Butyl-, *n*-Pentyl-, 2-Pentyl-, *neo*-Pentyl-, *n*-Hexyl- und 2-Hexylgruppe genannt. Ganz besonders bevorzugt ist die *n*-Butylgruppe.

Weitere bevorzugte Ausführungsformen des erfmdungsgemäßen Verfahren sind Verfahren, wobei:
(C) man als Verdünnungsmittel einen aromatischen Kohlenwasserstoff, vorzugsweise Benzol, Toluol oder Xylol, insbesondere Toluol, oder einen gegebenenfalls halogenierten aliphatischen Kohlenwasserstoff, vorzugsweise Cyclohexan, Methylcyclohexan, Dichlormethan, Chloroform, Tetrachlorkohlenstoff oder Dichlorethan, insbesondere Dichlormethan oder einen Ether, vorzugsweise Tetrahydrofuran (THF), Diethylether, Diisopropylether, *tert*-Butylmethylether (TBME) oder 1,2-Dimethoxyethan (DME), insbesondere 1,2-Dimethoxyethan verwendet;
(D)man die Umsetzung in einem Temperaturbereich von -10 °C bis + 90 °C, vorzugsweise von 0 °C bis 80 °C, insbesondere von 20 bis 65 °C durchfuhrt;
(E) man 0,75 bis 100 Äquivalente, vorzugsweise 1,5 bis 5,5 Äquivalente, insbesondere etwa 4 Äquivalente Ethylbromid bezogen auf 1 Äquivalent einer Verbindung der Formel (II) einsetzt;
(F) man 2,5 bis 100 Äquivalente, vorzugsweise 3,8 bis 10,5 Äquivalente, insbesondere 7,5 bis 8,5 Äquivalente an Base bezogen auf 1 Äquivalent einer Verbindung der Formel (II) einsetzt;
(G) man 0,01 bis 0,5 Äquivalente, vorzugsweise 0,02 bis 0,2 Äquivalente, insbesondere 0,05 bis 0,15 Äquivalente an Phasentransferkatalysator bezogen auf 1 Äquivalent einer Verbindung der Formel (II) einsetzt;
(H) man nach Beendigung der Umsetzung Wasser zur Reaktionsmischung hinzufügt, die Phasen trennt, die organische Phase mit Wasser wäscht, unter reduziertem Druck einengt und den Rückstand mit einer Säure behandelt und das erhaltene Säureadditionssalz isoliert;
(I) man den erhalten Wirkstoff der Formel (I) mit einer anorganischen oder organischen Säure behandelt. Die resultierenden Säureadditions-Salze sind beispielsweise Hydrochloride, Hydrobromide, Phosphate, Nitrate, Perchlorate, Sulfate, Citrate, Lactate, Tartrate, Maleate, Fumarate, Mandelate, Benzoate, Ascorbate, Cinnamate, Benzolsulfonate, Methansulfonate, Stearate, Succinate, Glutamate, Glycolate, Toluolp-sulfonate, Formate, Malonate, Naphthalin-2-sulfonate, Salicylate und Acetate. Besonders bevorzugt sind die Citrate. Solche Salze werden entsprechend bekannter Herstellmethoden hergestellt.

In einer besonders bevorzugten Ausführungsform werden 4 Äquivalente Ethylbromid, gegebenenfalls gelöst in 1,2-Dimethoxyethan, zu einem Gemisch aus 1 Äquivalent einer Verbindung der Formel (II), der etwa 20 fachen Gewichtsmenge an 1,2-Dimethoxyethan bezogen auf (II), etwa 8 Äquivalenten KOH, und etwa 0,1 Äquivalenten Tetra-n-butylammonium Hydrogensulfat innerhalb von 5 bis 60 Minuten bei einer Temperatur zwischen 20 und 35 °C unter Rühren gegeben. Nach Beendigung der Zugabe wird 30 bis 300, vorzugsweise etwa 45 bis 180 Minuten bei einer Temperatur zwischen 40 und 80 °C nachgerührt. Anschließend wird Wasser hinzugefügt, und bei der angegebenen Temperatur weitere 30 bis 300, vorzugsweise etwa 45 bis 180 Minuten gerührt, danach wird die organische Phase abgetrennt. Die organische Phase wird eingeengt und der Rückstand mit einer Säure, vorzugsweise Zitronensäure behandelt.

Das Säureadditionssalz der Verbindung der Formel (I) wird isoliert und getrocknet.

Weitere vorteilhafte Aspekte der erfindungsgemäßen Vorgehensweise sind die hohe Raum-Zeit-Ausbeute beim vorliegenden Prozess sowie die hohe Ausbeute und Reinheit der ohne weitere Aufreinigungsprozeduren erhaltenen Verbindung der Formel (I) bzw. ihres Salzes.

Die nachfolgenden Beispiele dienen der Illustration exemplarisch durchgeführter Verfahren zur Herstellung einer Verbindung der Formel (I).

### Beispiel 1 (1R,2R,3S)-2-Ethoxymethyl-3-(3,4-dichlorophenyl)-tropan Citrat

Zu einem Gemisch aus 10 g (0,0333 mol) (1R,2R,3S)-2-Hydroxymethyl-3-(3,4-dichlorophenyl)-tropan (hergestellt entsprechend WO 97/30997), 14,92 g gepulvertem (0,266 Mol KOH) Ätzkali, 1,16 g (0,00334 Mol) Tetra-*n*-butylammonium Hydrogensulfat und 200 ml DME werden bei einer Temperatur zwischen 20 und 31 °C unter Rühren 14,6 g (0,134 Mol) Ethylbromid innerhalb von 15 Minuten gegeben.

Nach Beendigung der Zugabe wird 1,5 Stunden bei einer Temperatur zwischen 58 und 62 °C nachgerührt. Anschließend werden 76.ml Wasser hinzugefügt, eine weitere Stunde bei dieser Temperatur gerührt und die organische Phase wird abgetrennt. Die organische Phase wird am Rotationsverdampfer unter reduziertem Druck eingeengt. Der Rückstand wird mit 90 ml Aceton bei 55 °C gelöst, filtriert und mit 10 ml Aceton nachgespült. Die erhaltene Lösung wird mit einem Gemisch von 6,4 g (0,0333 Mol) Citronensäure und 20 ml Methanol bei 40 °C behandelt. Die Kristallsuspension wird auf 20 °C abgekühlt und eine Stunde bei 15 bis 20 °C nachgerührt. Die erhaltenen Kristalle werden isoliert und mit 33 ml Aceton gewaschen. Nach dem Trocknen im Vakuumtrockenschrank bei 40 °C erhält man 14,55 g (83,6 % d. Th.) der Titelverbindung als gelbliche Kristalle (Reinheit über 99,4 %).

### Beispiel 2 (1R,2R,3S)-2-Ethoxymethyl-3-(3,4-dichlorophenyl)-tropan Citrat

Zu einem Gemisch aus 12 g (0,0400 mol) (1R,2R,3S)-2-Hydroxymethyl-3-(3,4-dichlorophenyl)-tropan (hergestellt entsprechend WO 97/30997), 17,9 g gepulvertem (0,320 Mol KOH) Ätzkali, 1,39 g (0,00409 Mol) Tetra-*n*-butylammonium Hydrogensulfat und 220 ml DME werden bei einer Temperatur zwischen 20 und 31 °C unter Rühren 17,5 g (0,161 Mol) Ethylbromid in 20 ml 1,2-Dimethoxyethan gelöst innerhalb von 15 Minuten gegeben.

Nach Beendigung der Zugabe wird 1,5 Stunden bei einer Temperatur zwischen 58 und 62 °C nachgerührt. Anschließend werden 76 ml Wasser hinzugefügt, eine weitere Stunde bei dieser Temperatur gerührt und die organische Phase wird abgetrennt. Die organische Phase wird am Rotationsverdampfer unter reduziertem Druck eingeengt. Der Rückstand wird mit 108 ml Aceton bei 55 °C gelöst, filtriert und mit 40 ml Aceton nachgespült. Die erhaltene Lösung wird mit einem Gemisch von 7,68 g (0,0400 Mol) Citronensäure und 24 ml Methanol bei 40 °C behandelt. Die Kristallsuspension wird auf 20 °C abgekühlt und eine Stunde bei 15 bis 20 °C nachgerührt. Die erhaltenen Kristalle werden isoliert und mit mind. 80 ml Aceton gewaschen. Nach dem Trocknen im Vakuumtrockenschrank bei 40 °C erhält man 17,44 g (83,85 % d. Th.) der Titelverbindung als gelbliche Kristalle (Reinheit über 99,5 %).

### Vergleichsbeispiel 1 (1R,2R,3S)-2-Ethoxymethyl-3-(3,4-dichlorophenyl)-tropan Citrat

(gemäß WO 97/30997)

Zu einem Gemisch aus (1R,2R,3S)-2-hydroxymethyl-3-(3,4-dichlorophenyl)tropan (26,9 g, 0,09 Mol) und THF (200 ml) werden Natriumhydrid 60% in Öl (4,6 g, 0,12 Mol) and Ethylsulfat (15,7 ml, 0,12 mol) gegeben und eine halbe Stunde auf 30-40 ° C erhitzt. Die Reaktionsmischung wird bei Raumtemperatur über Nacht weitergerührt, anschließend eine Stunde auf 30-40 ° C erhitzt und in 500 ml Wasser gegossen. Die Mischung wird zweimal mit TBDME extrahiert, die organischen Phasen werden mit Wasser gewaschen und über MgSO₄ getrocknet. Man erhält 32,82 g der Base.

Zu einer Lösung des so erhaltenen (1R,2R,3S)-2-Ethoxymethyl-3(3,4-dichlorophenyl)tropans in 96%-igem Ethanol (275 ml) wird Zitronensäure (19.2 g, 0.1 mol) gegeben. Die Lösung wird unter Rückfluss erhitzt und 3 Stunden bei Raumtemperatur zur Kristallisation stehen gelassen. Die Mischung wird eine halbe Stunde auf ein Eisbad gestellt, das kristalline Produkt wird abfiltriert und mit 96%-igem Ethanol (50 ml und 25 ml) gewaschen. Nach dem Trocknen erhält man 32,85 g (70% d. Th.) der Titelverbindung mit einem Schmelzpunkt von 153-155,5 °C.

## Patentansprüche

1. Verfahren zur Herstellung eines 2-(Ethoxymethyl)-tropanderivates der Formel (I) oder einem pharmazeutisch akzeptablen Salz davon, 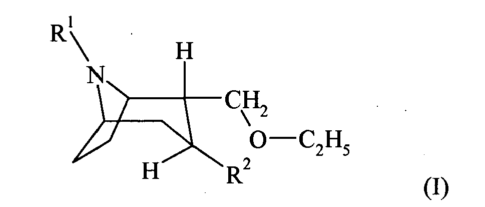 worin
R¹ für Wasserstoff oder C₁₋₆ Alkyl steht; und
R² für gegebenenfalls ein oder mehrfach durch Halogen, Trifluormethyl oder Cyano substituiertes Phenyl steht;
**dadurch gekennzeichnet, dass** man ein 2-(Hydroxymethyl)-tropanderivates der Formel (II), 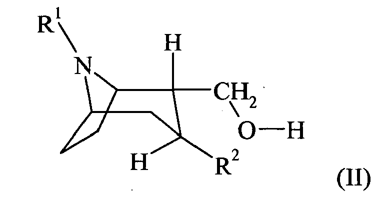 worin R¹ und R² die für Formel (I) angegebene Bedeutung aufweisen,
mit Ethylbromid in Gegenwart einer Base, eines Phasentransferkatalysators, und gegebenenfalls einem Verdünnungsmittel umsetzt, und anschließend gegebenenfalls mit einer Säure behandelt; und worin man als Base Lithiumhydroxid, Natriumhydroxid oder Kaliumhydroxid einsetzt.

2. Verfahren nach Anspruch 1, worin
R¹ für Methyl steht, und
R² für 3,4-Dichlorphenyl steht.

3. Verfahren nach Anspruch 1 oder 2, **dadurch gekennzeichnet, dass** man als Base gepulvertes Kaliumhydroxid einsetzt.

4. Verfahren nach einem der Ansprüche 1 bis 3, **dadurch gekennzeichnet, dass** man als Phasentransferkatalysator ein Tetraalkylammonium- oder Tetraalkylphosphöniumsalz verwendet.

5. Verfahren nach Anspruch 4, **dadurch gekennzeichnet, dass** man als Phasentransferkatalysator ein Tetraalkylammonium Hydrogensulfat verwendet.

6. Verfahren nach einem der Ansprüche 1 bis 5, **dadurch gekennzeichnet, dass** man als Verdünnungsmittel einen aromatischen Kohlenwasserstoff, einen gegebenenfalls halogenierten aliphatischen Kohlenwasserstoff oder einen Ether verwendet.

7. Verfahren nach Anspruch 6, **dadurch gekennzeichnet, dass** man als Verdünnungsmittel Toluol, Dichlormethan oder 1,2-Dimethoxyethan verwendet.

8. Verfahren nach einem der Ansprüche 1 bis 7, **dadurch gekennzeichnet, dass** man die Umsetzung in einem Temperaturbereich von -10 °C bis + 90 °C durchführt.

9. Verfahren nach einem der Ansprüche 1 bis 8, **dadurch gekennzeichnet, dass** man das Ethylbromid innerhalb von 5 bis 180 Minuten zudosiert und die erhaltene Reaktionsmischung noch weitere 30 bis 180 Minuten nachrührt.

10. Verfahren nach einem der Anspruch 1 bis 9, **dadurch gekennzeichnet, dass** man 0,75 bis 100 Äquivalente Ethylbromid bezogen auf 1 Äquivalent der Formel (II) einsetzt.

11. Verfahren nach einem der Ansprüche 1 bis 10, **dadurch gekennzeichnet, dass** man 2,5 bis 100 Äquivalente an Base bezogen auf 1 Äquivalent der Formel (II) einsetzt.

12. Verfahren nach einem der Ansprüche 1 bis 11, **dadurch gekennzeichnet, dass** man 0,01 bis 0,5 Äquivalente an Phasentransferkatalysator bezogen auf 1 Äquivalent der Formel (II) einsetzt.

13. Verfahren zur Herstellung eines Salzes eines 2-(Ethoxymethyl)-tropanderivates der Formel (I) nach einem der Ansprüche 1 bis 12, **dadurch gekennzeichnet, dass** man nach Beendigung der Umsetzung der Verbindung der Formel (II) mit Ethylbromid Wasser zur Reaktionsmischung hinzufügt, die Phasen trennt, die organische Phase mit Wasser wäscht, unter reduziertem Druck einengt und den Rückstand ohne weitere Aufreinigung mit der entsprechenden Säure behandelt.

## Claims

1. Process for the preparation of a 2-(ethoxymethyl)-tropane derivative of formula (I) or a pharmaceutically acceptable salt thereof, 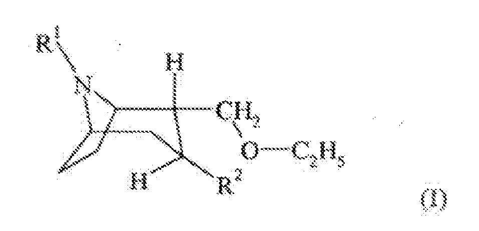 where
R¹ represents hydrogen or C₁₋₆ alkyl; and
R² represents phenyl which is optionally substituted one or more times with halogen, trifluoromethyl or cyano;
**characterised in that** a 2-(hydroxymethyl)-tropane derivative of formula (II) 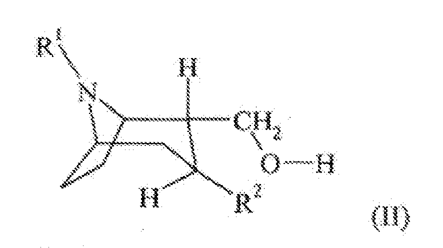 where R¹ and R² have the meaning specified for formula (I) is reacted with ethyl bromide in the presence of a base, a phase transfer catalyst, and optionally a diluent, and subsequently optionally treated with an acid;
and wherein lithium hydroxide, sodium hydroxide or potassium hydroxide is used as a base.

2. Process according to claim 1, wherein R¹ represents methyl, and R² represents 3,4-dichlorophenyl.

3. Process according to either claim 1 or claim 2, **characterised in that** powdered potassium hydroxide is used as a base.

4. Process according to any one of claims 1 to 3, **characterised in that** a tetraalkylammonium salt or tetraalkylphosphonium salt is used as the phase transfer catalyst.

5. Process according to claim 4, **characterised in that** a tetraalkylammonium hydrogen sulphate is used as the phase transfer catalyst.

6. Process according to any one of claims 1 to 5, **characterised in that** an aromatic hydrocarbon, an optionally halogenated aliphatic hydrocarbon or an ether is used as a diluent.

7. Process according to claim 6, **characterised in that** toluene, dichloromethane or 1,2-dimethoxyethane is used as a diluent.

8. Process according to any one of claims 1 to 7, **characterised in that** the reaction is carried out in a temperature range from -10 °C to +90 °C.

9. Process according to any one of claims 1 to 8, **characterised in that** the ethyl bromide is metered within 5 to180 minutes and the reaction mixture obtained is subsequently stirred for a further 30 to 180 minutes.

10. Process according to any one of claims 1 to 9, **characterised in that** 0.75 to 100 equivalents of ethyl bromide are used based on 1 equivalent of formula (11).

11. Process according to any one of claims 1 to 10, **characterised in that** 2.5 to 100 equivalents of base are used based on 1 equivalent of formula (II).

12. Process according to any one of claims 1 to 11, **characterised in that** 0.01 to 0.5 equivalents of phase transfer catalyst are used based on 1 equivalent of formula (II).

13. Process for the preparation of a salt of a 2-(ethoxymethyl)-tropane derivative of formula (I) according to any one of claims 1 to 12, **characterised in that** after the reaction of the compound of formula (II) with ethyl bromide is complete, water is added to the reaction mixture, the phases are separated, the organic phase is washed with water and concentrated under reduced pressure, and the residue is treated with the corresponding acid without further purification.

## Revendications

1. Procédé de préparation d'un dérivé de 2-(éthoxyméthyl)-tropane de formule (I) ou d'un sel pharmaceutiquement acceptable de celui-ci, 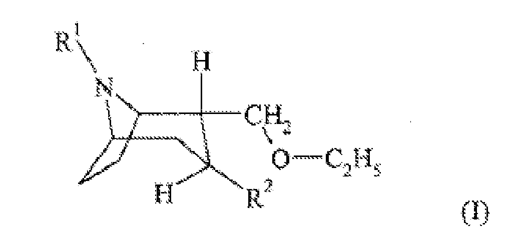 dans laquelle
R¹ est un atome d'hydrogène ou un groupe alkyle en C₁ à C₆ ; et
R² est un groupe phényle éventuellement substitué une ou plusieurs fois par un halogène, un groupe trifluorométhyle ou cyano ;
**caractérisé en ce que** l'on fait réagir un dérivé de 2-(hydroxyméthyl)-tropane de formule (II) 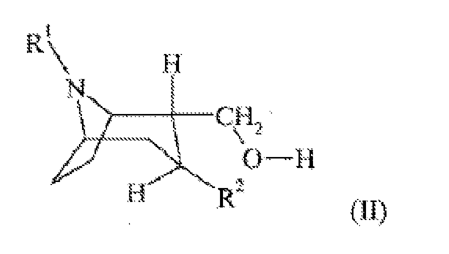 dans laquelle R¹ et R² ont la signification indiquée pour la formule (I),
avec du bromure d'éthyle en présence d'une base, d'un catalyseur de transfert de phase et éventuellement d'un diluant, et dans lequel ensuite, on traite éventuellement avec un acide ; et de l'hydroxyde de lithium, de l'hydroxyde de sodium ou de l'hydroxyde de potassium est utilisé comme base.

2. Procédé selon la revendication 1, dans lequel
R¹ est un groupe méthyle, et
R² est un groupe 3,4-dichlorophényle.

3. Procédé selon la revendication 1 ou 2, **caractérisé en ce que** l'on utilise comme base de l'hydroxyde de potassium pulvérisé.

4. Procédé selon l'une des revendications 1 à 3, **caractérisé en ce que** l'on utilise comme catalyseur de transfert de phase, un sel de tétraalkylammonium ou de tétraalkylphosphonium.

5. Procédé selon la revendication 4, **caractérisé en ce que** l'on utilise comme catalyseur de transfert de phase, un hydrogénosulfate de tétraalkylammonium.

6. Procédé selon l'une des revendications 1 à 5, **caractérisé en ce que** l'on utilise comme diluant, un hydrocarbure aromatique, un hydrocarbure aliphatique éventuellement halogéné ou un éther.

7. Procédé selon la revendication 6, **caractérisé en ce que** l'on utilise comme diluant, du toluène, du dichlorométhane ou du 1,2-diméthoxyéthane.

8. Procédé selon l'une des revendications 1 à 7, **caractérisé en ce que** l'on effectue la réaction dans une plage de températures de -10° C à + 90° C.

9. Procédé selon l'une des revendications 1 à 8, **caractérisé en ce que** l'on ajoute le bromure d'éthyle en 5 à 180 minutes et l'on agite le mélange réactionnel obtenu pendant encore 30 à 180 minutes.

10. Procédé selon l'une des revendications 1 à 9, **caractérisé en ce que** l'on utilise 0,75 à 100 équivalents de bromure d'éthyle par rapport à 1 équivalent de formule (II).

11. Procédé selon l'une des revendications 1 à 10, **caractérisé en ce que** l'on utilise 2,5 à 100 équivalents de base par rapport à 1 équivalent de formule (II).

12. Procédé selon l'une des revendications 1 à 11, **caractérisé en ce que** l'on utilise 0,01 à 0,5 équivalents de catalyseur de transfert de phase par rapport à 1 équivalent de formule (II).

13. Procédé de préparation d'un sel d'un dérivé de 2-(éthoxyméthyl)-tropane de formule (I) selon l'une des revendications 1 à 12, **caractérisé en ce que** l'on ajoute de l'eau au mélange réactionnel après la fin de la réaction du composé de formule (II) avec le bromure d'éthyle, on sépare les phases, on lave la phase organique à l'eau, on la réduit par évaporation sous pression réduite et on traite le résidu sans autre purification avec l'acide correspondant.
